# EUROPÄISCHE PATENTSCHRIFT

(11) **EP 2 741 604 B1**
(45) Veröffentlichungstag und Bekanntmachung des Hinweises auf die Patenterteilung: **26.08.2015**
(21) Anmeldenummer: 12737297.7
(22) Anmeldetag: 23.07.2012
(51) Int. Cl.: A01K 61/00, A23K 1/00, A23K 1/06, A23K 1/18, A01K 67/033

(54) **VERFAHREN ZUR VERWERTUNG VON VINASSE**
METHOD FOR USING VINASSE
PROCÉDÉ POUR LA VALORISATION DE LA VINASSE

(30) Priorität: 09.08.2011 WO PCT/EP2011/063706; 26.03.2012 DE 102012204807; 24.04.2012 DE 102012206700
(43) Veröffentlichungstag der Anmeldung: 18.06.2014
(73) Patentinhaber: Weber Ultrasonics GmbH, 76307 Karlsbad-Ittersbach (DE)
(72) Erfinder: VOGEL, Siegmar, 12167 Berlin (DE)
(74) Vertreter: Lemcke, Brommer & Partner Patentanwälte Partnerschaft mbB
(86) Internationale Anmeldenummer: PCT/EP2012/064367
(87) Internationale Veröffentlichungsnummer: WO 2013/020797

(56) Entgegenhaltungen:
- WO-A1-2008/116278
- WO-A1-2009/093091
- WO-A2-01/50880
- WO-A2-2004/047523
- US-A- 4 342 650
- US-A- 4 357 358

## Beschreibung

Die vorliegende Erfindung betrifft ein Verfahren nach dem Oberbegriff des Patentanspruchs 1 zur Produktion von Futtermittel aus Krebstieren der Gattung Artemia oder Copepoda (Ruderfußkrebse) oder Daphnia (Wasserflöhe), aus Rädertierchen (Rotifera) oder aus Eukaryonten (Protozoen).

Weiterhin betrifft die Erfindung ein Verfahren nach dem Oberbegriff des Patentanspruchs 2 zur Verarbeitung von Vinasse, insbesondere im Zuge der Bioethanolgewinnung.

Außerdem betrifft die vorliegende Erfindung ein Verfahren nach dem Oberbegriff des Patentanspruchs 16 zur Produktion von Futtermittel aus Krebstieren der Gattung Artemia oder Copepoda (Ruderfußkrebse) oder Daphnia (Wasserflöhe), aus Rädertierchen (Rotifera) oder aus Eukaryonten (Protozoen) und/oder zur Produktion von Rohstoffen aus Algen.

Bei Vinasse handelt es sich um Fermentationsreste als Nebenprodukt bei der Bioethanolproduktion, welche sich in den vergangenen Jahrzehnten zu einem weltweiten Umweltproblem entwickelt hat. Die regelmäßig erfolgende unbehandelte Entsorgung der Vinasse in Flüsse und in der Landwirtschaft hat aufgrund von deren chemischer Zusammensetzung und der biologischen Belastung zu enormen Umweltzerstörungen geführt. Hierzu gehört unter anderem lokale Fischsterben und eine Übersäuerung oder Überdüngung von Agrarböden durch das unkontrollierte Verklappen der Vinasse. Das Problem ist umso ernsthafter, da bei der Ethanolproduktion je nach Ausgangsmaterial zwischen 4 und 15 I Dünnvinasse auf jeden produzierten Liter Bioethanol erzeugt werden.

Die Ethanolindustrie sucht intensiv nach Lösungsansätzen, um das Vinasseproblem in den Griff zu bekommen. So wird heute in vielen Ländern der Welt die erzeugte Vinasse durch energieintensives Verdampfen von Flüssigstoffen eingedickt, um so die Kosten des Weitertransports zu verringern. Das Endprodukt kann anschließend als Tierfutterergänzung oder als Düngemittel eingesetzt werden. Die hierbei anfallenden zusätzlichen Kosten sind enorm. Zudem bleibt der große Prozesswärmegehalt der Vinasse, deren Temperatur bei Entstehung etwa 60°C beträgt, ungenutzt.

Aus der WO 2004 047 523 ist die Verwendung von Melasse als Futtermittel für Krebstiere bekannt.

Der Erfindung liegt die Aufgabe zugrunde, alternative Verfahren zur Verwertung von Vinasse anzugeben, um der vorstehend beschriebenen Umweltproblematik Herr zu werden und die bislang mit der Entsorgung von Vinasse verbundenen Transport- und Energiekosten zu reduzieren. Außerdem soll ein nachhaltiger Beitrag zum Thema Ernährung, insbesondere auch in Schwellen- und Entwicklungsländern, geleistet werden.

Die Aufgabe wird erfindungsgemäß gelöst durch ein Verfahren mit den Merkmalen des Patentanspruchs 1, durch ein Verfahren mit den Merkmalen des Patentanspruchs 2 sowie durch ein Verfahren mit den Merkmalen des Patentanspruchs 16. Vorteilhafte Weiterbildungen dieser Verfahren sind jeweils Gegenstand von Unteransprüchen, deren Wortlaut hiermit durch ausdrückliche Bezugnahme in die Beschreibung aufgenommen wird, um unnötige Textwiederholungen nach Möglichkeit zu vermeiden.

Ein erfindungsgemäßes Verfahren zur Produktion von Futtermittel aus Krebstieren der Gattung Artemia oder der Gattung Copepoda (Ruderfußkrebse) oder der Gattung Daphnia (Wasserflöhe), aus Rädertierchen (Rotifera) oder aus Eukaryonten (Protozoen) zeichnet sich dadurch aus, dass die Artemia/Copepoda/Daphnia/Rotifera/Protozoa-Kulturen zumindest teilweise mit Vinasse, vorzugsweise Dünnvinasse, insbesondere aus der Bioethanolproduktion, und den darin enthaltenen Hefezellen gefüttert werden. Die gezüchteten Krebstiere bzw. Rotifera/Protozoa lassen sich anschließend zu Futtermittel insbesondere für die Fischzucht verwenden, beispielsweise nach entsprechender Verarbeitung (Pelletierung).

Ein anderes erfindungsgemäßes Verfahren zur Verarbeitung von Vinasse, insbesondere im Zuge der Bioethanolgewinnung, zeichnet sich dadurch aus, dass die Vinasse, vorzugsweise nach entsprechender Aufbereitung, als Futter für Artemia/Copepoda/Daphnia/Rotifera/Protozoa-Kulturen und/oder für Algenkulturen verwendet wird, um die in der Vinasse enthaltenen Hefezellen zumindest teilweise zu entfernen. Ein Verklappen von Vinasse lässt sich auf diese Weise vermeiden.

Ein wieder anderes erfindungsgemäßes Verfahren zur Produktion von Futtermittel aus Krebstieren der Gattung Artemia oder der Gattung Copepoda oder der Gattung Daphnia, aus Rädertierchen (Rotifera) oder aus Eukaryonten (Protozoen) und/oder zur Produktion von Rohstoffen aus Algen zeichnet sich dadurch aus, dass bei der Vinasseproduktion, insbesondere im Zuge der Bioethanolgewinnung, erzeugte Prozesswärme zum Erwärmen von Wasser oder dergleichen für die Artemia-, für die Copepoda-, für die Daphnia-, für die Rotifera-, für die Protozoa- und/oder für die Algenzucht verwendet wird. Damit wird die Zucht auch dort ermöglicht, wo klimatische Bedingungen ansonsten ungeeignet wären.

Auf diese Weise wird im Rahmen der vorliegenden Erfindung erstmals ein Gesamtkonzept vorgeschlagen, welches auf eine Entsorgung von Vinasse im herkömmlichen Sinne verzichtet und diese als in jeder Hinsicht wertvollen Rohstoff betrachtet, der sich auf vielfältige Art und Weise, vordringlich jedoch für die Erzeugung von Nährstoffen für bzw. in Aqua-Kulturen nutzen lässt, wodurch die vorstehend skizzierte Umweltproblematik weitestgehend beseitigt wird.

Einerseits ist im Rahmen der vorliegenden Erfindung vorgesehen, die Vinasse praktisch unmittelbar als Futtermittel für Artemia-/Copepoda-/Daphnia-/Rotifera-/Protozoa- Kulturen und/oder für Algenkulturen zu verwenden. Um diese nicht zu gefährden, kann in diesem Zusammenhang vorgesehen sein, den pH-Wert der Vinasse von dem Verfüttern anzuheben und vorzugsweise zusätzlich zu puffern. Dies kann insbesondere durch Zugabe von Carbokalk (CaCO₃) erfolgen. Als Alternative zu Carbokalk können auch Ca(OH)₂ oder andere Basen verwendet werden.

Ein anderer Aspekt der vorliegenden Erfindung betrifft dementsprechend ein Verfahren zur Verarbeitung von Vinasse, welches sich dadurch auszeichnet, dass die Vinasse als Futter für Artemia-/Copepoda-/Daphnia-/Rotifera-/Protozoa-Kulturen und/oder für Algenkulturen verwendet wird, was eine überaus umweltverträgliche Art der Verwertung/Entsorgung von Vinasse darstellt.

Um den im Zuge der Bioethanolproduktion anfallenden großen Vinassemengen von bis zu 50 m³ pro Stunde im Falle einer typischen Anlage Herr werden zu können, sieht eine bevorzugte Weiterbildung der erfindungsgemäßen Verfahren vor, dass die Artemia/Copepoda/Daphnia/Rotifera/Protozoa in geschlossenen Intensivkulturen gehalten werden. Alternativ ist aber auch eine Zucht in halboffenen oder offenen Kulturen möglich.

Artemia hat sich aufgrund seiner Nährwertcharakteristiken in den vergangenen Dekaden als hervorragendes Futtermittel sowohl bei Süßwasser- als auch bei Salzwasser-Fischkulturen erwiesen. Aufgrund des steigenden Bedarfs an Artemia haben sich auch die Marktpreise auf hohem Niveau stabilisiert. Der wachsende Markt für Aqua-Kulturen benötigt neue Konzepte und Strategien in der Fütterung des Besatzes, wie vorliegend vorgeschlagen. Insbesondere reicht die weltweite Produktion an Bioethanol bzw. Vinasse aus, um den weltweiten Proteinbedarf bei Aquakulturen, einschließlich aller Wachstumseinschätzungen im Aquakultursektor, zu decken. Damit wäre dem Überfischen der Weltmeere ein deutlicher Schritt entgegengesetzt, und die Produktion von Fischmehl könnte zumindest stark eingeschränkt werden. In der Vergangenheit wurden in diesem Zusammenhang vielfach Pellets aus Fischmehlfabriken verwendet. Die hiermit zusammenhängenden großen ökologischen Problemen, wie z. B. die Überfischung der Meere und die Verarbeitung viel zu kleinen Rohmaterials von juvenilen Meeresbewohnern, sind hinreichend bekannt und verlangen nach einem generellen Umdenken bei den Betreibern derartiger Aqua-Kulturen.

Artemia ist aufgrund seiner biologischen Charakteristiken (hoher Multiplikationsfaktor) und der Möglichkeit einer Haltung in Intensivkultur eine Variante, die aufgrund mangelnder andersartiger technischer Ansätze bisher meist nur in offenen Systemen und nur in klimatologisch warmen Gegenden verbreitet ist. Zudem ist die Fütterung von Intensivkulturen von Artemia bislang ein nicht zu vernachlässigender Kostenfaktor.

Entsprechendes gilt sinngemäß für Copepoda (Ruderfußkrebse), welche eine gute Reproduzierbarkeit bei Fütterung mit Vinasse zeigen. Dies gilt sowohl für pelagische (Freiwasser-) als auch für benthische (auf und im Boden eines Gewässers lebende) Arten von Copepoden. Copepoden bilden die artenreichste Gruppe der Crustaceen (Krebstiere) und stellen den massemäßig größten Anteil des marinen Planktons (Zooplankton) dar, sodass sie für die Verwendung als Fisch- oder Garnelenfutter prädestiniert sind. Zudem existieren umfangreiche Studien über die Verwendung von Copepoden als Aufzuchtfutter in der Fischindustrie, wobei der hohe Nährwert besonders betont wird.

Auch Daphnien (Wasserflöhe), insbesondere Daphnia magna (Großer Wasserfloh), Rotifera und Protozoa lassen sich nach Untersuchungen der Anmelderin bei Fütterung mit Vinasse gut kultivieren.

Durch die vorgeschlagene Verwendung von Vinasse als Futtermittel für Artemia-, Daphnia- und Copepoda-Intensivkulturen bzw. zur Aufzucht von Rotifera/Protozoa lassen sich somit neben den bereits mehrfach erwähnten Problemen bei der Verwertung/Entsorgung von Vinasse auch die vorstehend angesprochenen Probleme im Zusammenhang mit dem Betrieb von Artemia-/Daphnia-/Copepoda-/Rotifera-/Protozoa-Intensivkulturen umgehen bzw. beseitigen.

Aufgrund der erfindungsgemäß vorgeschlagenen Verwendung der Prozessabwärme bei der Vinasseerzeugung lassen sich die angesprochenen Artemia-/Copepoda-Intensivkulturen auch in solchen Klimazonen realisieren, in denen eine Artemia-, Daphnia-, Rotifera-, Protozoa- oder Copepodazucht sonst beispielsweise aufgrund zu niedriger Wassertemperaturen bzw. zu hohen Energiebedarfs nicht lukrativ wäre.

Zwar ist zu erwarten, dass sich die Verkaufspreise speziell für Artemia, Daphnia und Copepoda aber auch für Rotifera bzw. Protozoa im Zuge der vorgeschlagenen Intensivhaltung tendenziell nach unten entwickeln werden, gegenüber den ansonsten für die Vinasseentsorgung aufzuwendenden Kosten ergibt sich jedoch nach wie vor ein nicht zu vernachlässigender finanzieller Vorteil.

Im Rahmen der vorgeschlagenen Intensivhaltung von Artemia/Copepoda/Daphnia (Krebstiere) bzw. Rotifera/Protozoa (Kleinlebewesen) und/oder Algen sind Hochleistungsfiltrationsanlagen von Nöten, um potentiell gefährliche Keime im Prozesswasser abzutöten und zudem das Auftreten von Fäulnisprozessen zu verhindern, welche ansonsten insbesondere die Artemia-, Copepoda-, Daphnia-, Rotifera-, Protozoa- oder Algen-Kulturen gefährden könnten. In diesem Zusammenhang ist im Rahmen einer äußerst bevorzugten Weiterbildung der erfindungsgemäßen Verfahren vorgeschlagen, dass das in den Krebstier-Kulturen, den Kleinlebewesen-Kulturen und/oder den Algenkulturen verwendete (Prozess-)Wasser zur Entkeimung mit Ultraschall beaufschlagt wird, vorzugsweise mit Ultraschall im Megaschallbereich (f ≥ 500 kHz). Auf diese Weise lassen sich auch mikrobiologische Verunreinigungen des Prozesswassers entweder direkt beseitigen oder derart aufschließen, dass sie durch optional nachgeschaltete weitere Prozesswasserbehandlungsschritte leicht beseitigbar sind.

Eine andere Weiterbildung der erfindungsgemäßen Verfahren sieht in diesem Zusammenhang vor, dass das in den Krebstier/Kleinlebewesen-Kulturen und/oder der Algenkulturen verwendete Wasser zur Entkeimung alternativ oder zusätzlich mit kurzwelligem Licht bestrahlt wird, vorzugsweise im ultravioletten Spektralbereich. Höchst vorzugsweise wird hierzu Licht mit einer Wellenlänge im Bereich 1 bis 380 nm, vorzugsweise etwa 250 nm, verwendet.

Eine kontinuierliche Überwachung der Prozesswasserqualität der Krebstier-/Kleinlebewesen-/Algen-Intensivkulturen verbunden mit einer entsprechenden Automatisierung ermöglicht die Verarbeitung auch sehr großer Volumenströme, wie sie bei einem industriellen Verfahren zur Verwertung von Vinasse auftreten werden.

Vinasse lässt sich aufgrund ihrer Zusammensetzung nur schwer bzw. gar nicht filtrieren, was insbesondere an dem Vorhandensein großer Mengen an Hefezellen im Größenbereich von nur 5 bis 10 µm liegt. Die Verwendung von Nanofiltration oder Umkehrosmose ist bedingt durch die genannte organische Belastung mit Hefezellen nur unter Verwendung zusätzlicher Techniken durchführbar. Diese waren in der Vergangenheit nur durch aufwändige industrielle Prozesse zu realisieren. Die vorliegende Erfindung kann hier Abhilfe schaffen, indem sie das Abfallprodukt Vinasse der beschriebenen neuartigen Verwertung zuführt.

Die Anwendung und Nutzung mariner Algenkulturen bzw. der darin erfolgten Algen ist vielfältig und als solche nicht Gegenstand der vorliegenden Erfindung. Die erzeugten Algen können allerdings wiederum an Krebstier-/Kleinlebewesen-Kulturen verfüttert werden. Des Weiteren sind Verfahren bekannt geworden, mit denen aus Algen wertvolle Rohstoffe oder Treibstoffe, wie Biodiesel, oder sogar Wasserstoff gewonnen werden können. Alle diese Verfahren können im Zusammenhang mit der vorliegenden Erfindung Verwendung finden.

Algenkulturen stellen ebenso wie Krebstier-/Kleinlebewesen-Kulturen einen wachsenden Markt dar. Die Nutzung der Düngesalze aus der Vinasse in Produktionsanlagen für maritime Algen stellt somit eine wesentliche Verbesserung bei der Produktion von Meeresalgen dar und trägt zudem zur verbesserten Nutzung des gesamten Vinassepotentials bei.

Die Prozesswasser aus der genannten Algenproduktion können filtriert und mittels Umkehrosmose getrennt werden. Als besonders vorteilhaft hat sich herausgestellt, dass die hierbei entstehenden Salzlaugen in die Krebstier-/Kleinlebewesen-Produktion zurückgespeist werden können, um dort optimale Prozessparameter zu gewährleisten.

Zur Reinigung wird das Prozesswasser aus der Krebstier-/Kleinlebewesen-Produktion im Zuge einer besonderen Weiterbildung der Erfindung in Mikrofiltration über einen Membranfilter mit Porengröße vorzugsweise < 1 µm gereinigt. Grundsätzlich möglich ist die Verwendung von Poren der Größe 2 µm und kleiner. Der Membranfilter befindet sich dabei vorteilhafter Weise im Kulturbehälter und wird im outside-in-Verfahren betrieben. Er kann Bündel aus Membran-Hohlfasern umfassen, die innerhalb einer stabilen Halte- oder Rahmenkonstruktion angeordnet sind. Damit können die Membranen insgesamt aus dem Behälter entnommen werden, um sie zu reinigen. Die Reinigung erfolgt vorzugsweise durch Beaufschlagung mit Ultraschall.

Aufgrund der genannten Porengröße und der großen Filteroberfläche ist der Differentialdruck an der Filteroberfläche so gering, dass keine Gefahr für die Kleinstorganismen (insbesondere Artemia, Daphnia oder Copepoda aber auch Rotifera und Protozoa) besteht, angesaugt und verletzt zu werden.

In einem dem Membranfilter vorteilhafter Weise nachgeschaltetem Biofilter (biologisch aktiver Filter) werden im Permeat enthaltenes Ammonium und andere organische Moleküle bakteriell zersetzt. Zusätzlich oder alternativ kann das Prozesswasser (Permeat) auch als Dünger für die Algenproduktion eingesetzt werde.

Wie bereits angesprochen, besitzt die bei der Ethanolproduktion entstehende Vinasse eine Temperatur von etwa 60°C. Dieses Wärmepotential bleibt bislang ungenutzt, kann jedoch im Rahmen der vorliegenden Erfindung für den Krebstier-/Kleinlebewesen-Produktionsprozess sowie für die Algenzucht genutzt werden, welche jeweils Temperaturen von etwa 26°C erfordern. Damit erschließt sich der gesamte Markt auch in gemäßigten Zonen, so dass der Nahrungsbedarf von Aquakulturen ohne größeren Energie- und Kostenaufwand für den Transport auch in gemäßigten Zonen direkt insbesondere von Bioethanolwerken gedeckt werden kann.

Weitere Eigenschaften und Vorteile der vorliegenden Erfindung ergeben sich aus der nachfolgenden Beschreibung von Ausführungsbeispielen anhand der Zeichnung.
- Figur 1: zeigt eine schematische Darstellung des Prozessflusses betreffend den Gesamtprozess der im Rahmen der vorliegenden Erfindung vorgeschlagenen Vinassebehandlung;
- Figur 2: zeigt eine vereinfachte Darstellung des Gesamtprozesses gemäß Figur 1;
- Figur 3: zeigt eine spezielle Abwandlung des Verfahrens und des Prozessablaufs gemäß Figur 2;
- Figur 4: zeigt schematisch eine Krebstier-Produktionsanlage, wie sie bei der Durchführung des erfindungsgemäßen Verfahrens zum Einsatz kommen kann; und
- Figur 5: zeigt schematisch eine andere Krebstier-/Kleinlebewesen-Produktionsanlage, wie sie bei der Durchführung des erfindungsgemäßen Verfahrens zum Einsatz kommen kann.

Die nachfolgende Beschreibung bezieht sich exemplarisch auf die Verwertung/Entsorgung von Vinasse, wie sie als Nebenprodukt bei der Ethanolproduktion aus biologischen Rohstoffen, beispielsweise Zuckerrohr, anfällt. Diese Art der Ethanolproduktion wird im Rahmen der vorliegenden Beschreibung auch als "Bioethanolproduktion" bezeichnet. Die Erfindung ist jedoch grundsätzlich nicht auf die Verwendung derart erzeugter Vinasse beschränkt.

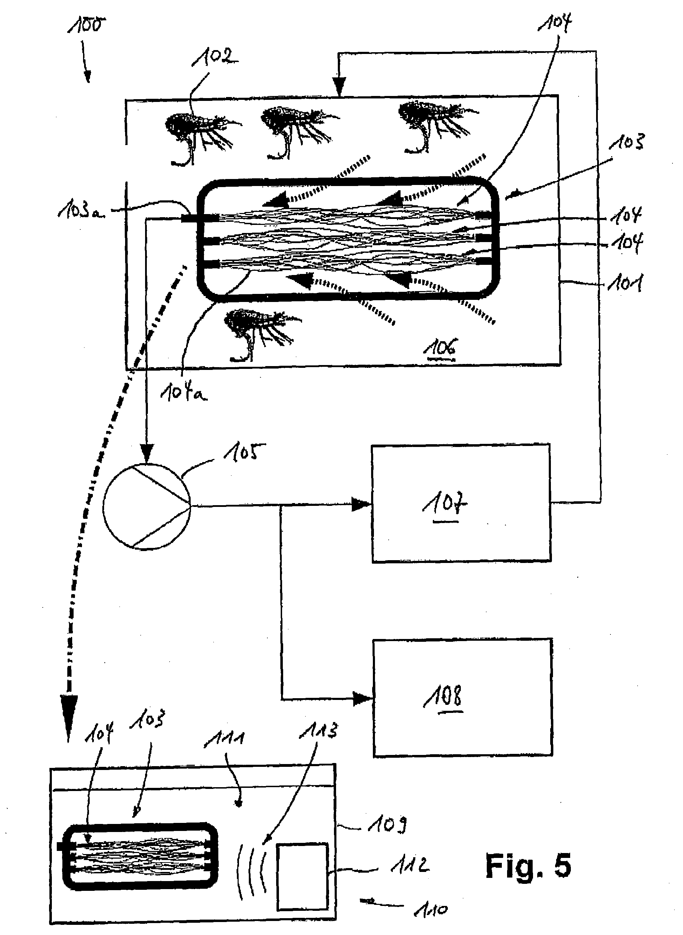

Des Weiteren beschränkt sich die nachfolgende Beschreibung größtenteils - mit Ausnahme der Figur 5 - auf die Zucht von Artemia. Die Erfindung ist jedoch grundsätzlich auch im Rahmen der Zucht von Krebstieren der Gattung Copepoda (Ruderfußkrebse) oder Daphnia (Wasserflöhe) und im Rahmen der Zucht von Rädertierchen (Rotifera) bzw. von Eukaryonten (Protozoa) oder allgemein Zooplankton anwendbar.

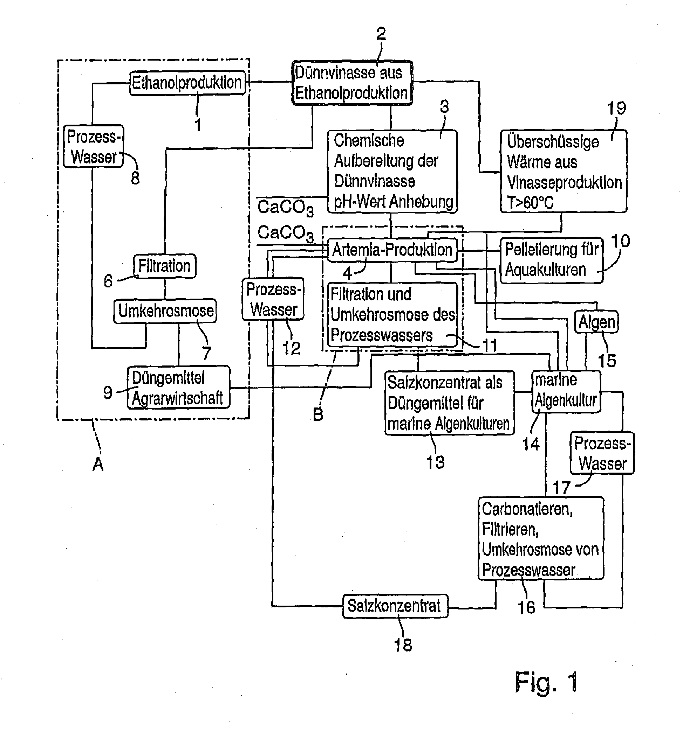

Figur 1 zeigt schematisch den Prozessfluss bei einem Gesamtprozess zur Vinassebehandlung (Entsorgung und Verwertung), wie er im Zuge der vorliegenden Erfindung realisierbar ist.

Bezugszeichen 1 bezeichnet die eigentliche Ethanolproduktion, bei der als Neben- oder Abfallprodukt Vinasse in Form von so genannter Dünnvinasse entsteht, was bei Bezugszeichen 2 angegeben ist. Die anfallende Vinassemenge ist relativ groß und kann bei einer typischen Anlage zur Produktion von Bioethanol aus Zuckerrohr bis zu 50 m³ pro Stunde betragen, was entsprechende Probleme bei der Entsorgung, jedoch auch entsprechende Möglichkeiten bei der Verwertung darstellt bzw. eröffnet.

Die vorliegende Erfindung schlägt nun vor, die Vinasse als Futtermittel für Artemia-Kulturen zu verwenden, um auf diese Weise aus den gezüchteten Artemia-Krebstieren Futtermittel zu gewinnen. Zu diesem Zweck wird die Vinasse bei Bezugszeichen 3 zunächst chemisch aufbereitet, wobei insbesondere ihr pH-Wert von typischerweise etwa 4,5 bis auf einen Wert von 8 angehoben wird, um die Artemia nicht zu gefährden. Nach der genannten Aufbereitung bei Bezugszeichen 3 wird die Vinasse bei Bezugszeichen 4 zur Artemia-Produktion verwendet, das heißt entsprechenden Artemia-Kulturen als Futtermittel beigegeben.

Bei dem in Verfahrensschritt 3 zugegebenen Zugabestoff zum Anheben des pH-Werts der Dünnvinasse kann es sich insbesondere um Carbokalk (CaCO₃) handeln. Das CaCO₃ kann - wie bereits ausgeführt - entweder in Schritt 3 zur chemischen Aufbereitung der Dünnvinasse oder in Schritt 4 direkt in der Artemia-Produktion verwendet werden - in letzterem Fall, um dort einer Übersäuerung des Aufzucht-Milieus effektiv entgegenzuwirken. Die Vinasse wird nach einer Filtration 6 und gegebenenfalls Umkehrosmose 7 in Prozesswasser 8 einerseits und Vinassesalze 9 andererseits geschieden, wobei die genannten Vinassesalze 9 anschließend insbesondere als Düngemittel in der Agrarwirtschaft Verwendung finden können. Das Prozesswasser 8 kann der Ethanolproduktion 1 wieder zugeführt werden, so dass sich insofern ein geschlossener Kreislauf ergibt. Die anfallenden Vinassesalze 9 lassen sich im Rahmen der Erfindung auch alternativ weiterverwerten, worauf weiter unten noch genauer eingegangen wird.

Der eigentliche Ertrag der Artemia-Produktion 4 in Form von geernteten Artemia-Krebstieren wird bei Bezugszeichen 10 zur Verwendung in Aquakulturen, beispielsweise für die Fischzucht, in Form von Pellets, Flocken oder dergleichen bereitgestellt. Wie der Fachmann erkennt, ist die Verwendung des Ertrags der Artemia-Produktion 4 jedoch keinesfalls auf die vorstehend anhand von Bezugszeichen 10 beispielhaft erläuterten Verwendungen beschränkt.

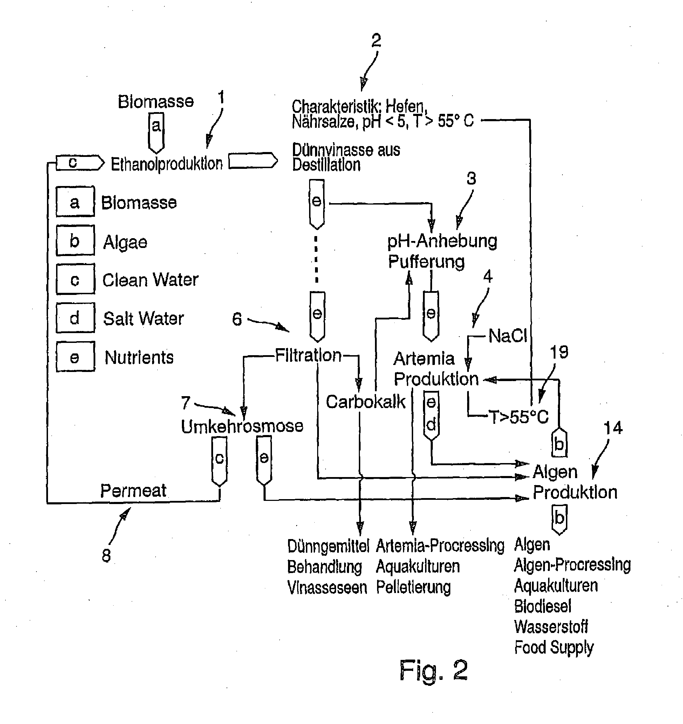

Die Gesamtanlage zur Artemia-Produktion gemäß Verfahrensschritt 4 einschließlich einer nachgeschalteten Filtration mit Umkehrosmose-Behandlung des Prozesswassers 11 ist in Figur 1 mit dem Bezugszeichen B (strichpunktierte Linie) gekennzeichnet und wird weiter unten anhand der Figur 2 noch näher beschrieben.

Das Prozesswasser selbst kann gemäß Verfahrensschritt 12 wieder der Artemia-Produktion 4 zugeführt werden.

Das bei der Umkehrosmose des Prozesswassers 11 der Artemia-Produktion 4 anfallende Salzkonzentrat 13 lässt sich - gegebenenfalls nach chemischer Aufbereitung - als Düngemittel für marine Algenkulturen 14 verwenden, welche als nutzbaren Ertrag entsprechende Algen 15 liefern, welche entweder direkt verwertet (z. B. für die Erzeugung von Biodiesel) oder im Rahmen der vorliegenden Erfindung wiederum der Artemia-Produktion 4 als Nährstoff zugeführt werden können. Wie weiter oben bereits angesprochen wurde, besteht auch die Möglichkeit, die im Rahmen des Verfahrens A anfallenden Düngemittelsalze 9 zum Düngen der marinen Algenkulturen 14 zu verwenden.

Im Zusammenhang mit den marinen Algenkulturen 14 besteht die Möglichkeit, die anfallenden flüssigen (Abfall-)Stoffe zu carbonatieren, zu filtrieren und per Umkehrosmose zu behandeln (Bezugszeichen 16), wobei insbesondere das Prozesswasser 17 wieder den Algenkulturen 14 zugeführt werden kann.

Das im Verfahrensschritt 16 bei der Umkehrosmose anfallende Salzkonzentrat 18 kann zusammen mit dem bereits angesprochenen Prozesswasser 16 wiederum der Artemia-Produktion 4 zugeführt werden, um den Artemia-Krebstieren optimale Wachstumsbedingungen zu garantieren.

Um den vorstehend beschriebenen Gesamtprozess gemäß Figur 1 abzurunden, kann im Rahmen der vorliegenden Erfindung weiterhin vorgesehen sein, dass die bei der Erzeugung von Dünnvinasse 2 im Rahmen der Ethanolproduktion entstehende Abwärme 19 mittels geeigneter Wärmetauscheinrichtungen im Rahmen der Artemia-Produktion 4 und/oder in den marinen Algenkulturen 14 nutzbar gemacht wird. Dies kann dadurch geschehen, dass beispielsweise Hallen oder Gebäude, in denen die genannten Kulturen angesiedelt sind, beheizt werden, um ein optimales Wachstumsumfeld zu schaffen. Es besteht zusätzlich oder alternativ auch die Möglichkeit, das eingesetzte Prozesswasser 12, 17 kostengünstig auf eine optimale Wachstumstemperatur zu erwärmen, indem hierfür die überschüssige Wärme 19 aus der Vinasseproduktion genutzt wird.

Anfallender Carbokalk kann zur Beseitigung vergangener "Umweltsünden" verwendet werden, wie zur Neutralisierung von so genannten Vinasseseen, in denen die bei der Ethanolproduktion anfallende Vinasse zwischengelagert bzw. entsorgt wurde, oder zur Neutralisierung übersäuerter Böden.

Eine bevorzugte Realisierung des vorstehend beschriebenen Gesamtprozesses zur Vinassebehandlung sieht vor, im Anschluss an den Verfahrensschritt 2 vor den Verfahrensschritten 3 und 6 eine Mengensteuerung oder Fraktionierung einzubauen, um die erzeugte Dünnvinasse wahlweise bzw. in einer ersten Menge der Filtration 6 und/oder in einer zweiten Menge der chemischen Aufbereitung in Schritt 3 zwecks anschließender Verwendung in der Artemia-Produktion 4 aufzuteilen - je nach momentanem Bedarf.

Figur 2 zeigt eine alternative, vereinfachte Darstellung des Gesamtprozesses aus Figur 1, wobei gleiche Bezugszeichen gleiche oder gleichwirkende Prozessschritte bezeichnet. Die Buchstaben "a" bis "e" bezeichnen Masseströme von Biomasse (a), Algen (engl. algae) (b), Frischwasser (clean water) (c), Salzwasser (salt water) (d) und Nährstoffen (nutrients) (e).

Gemäß Figur 2 entsteht aus der zugeführten Biomasse im Rahmen der Ethanolproduktion 1 wiederum Dünnvinasse 2, welche sich durch die in ihr enthaltenen Hefen und Nährsalze bei einem pH-Wert von weniger als 5 und einer Temperatur von mindestens 55°C oder darüber auszeichnet. Der bereits anhand von Figur 1 beschriebenen chemischen Aufbereitung in Schritt 3 (Anhebung des pH-Werts und entsprechende Pufferung) folgt das Zuführen der Vinasse zur Artemia-Produktion 4. Alternativ kann bei Bezugszeichen 6 eine Filtration gefolgt von einer Umkehrosmose-Behandlung 7 durchgeführt werden, woraufhin das aufbereitete Prozesswasser als Permeat 8 wieder der Ethanolproduktion 1 zugeführt wird. Der bei der Filtration 6 ggf. anfallende Carbokalk kann entweder bei Bezugszeichen 3 wiederverwertet oder als Düngemittel bzw. zur Behandlung von Vinasseseen eingesetzt werden.

Die Artemia-Produktion 4 liefert Futtermittel für Aquakulturen, beispielsweise für die Fischzucht, wie bereits beschrieben. Dies ist vorzugsweise mit einer Pelletierung oder einer sonstigen Verarbeitung der erzeugten Artemia verbunden, wie in Figur 2 angegeben.

Das aus der Artemia-Produktion 4 resultierende Salzwasser bzw. die verbleibenden (Vinasse-)Nährstoffe werden bei Bezugszeichen 14 zur Algenproduktion verwendet. Die erzeugten Algen können auf vielfältige Weise verwendet bzw. weiter verarbeitet werden, beispielsweise zur Erzeugung von Treibstoffen oder zur Herstellung von Nahrungsmitteln. Es sind sogar Algen-Kulturen bekannt, die zur Erzeugung von Wasserstoff eingesetzt werden können, was entsprechend ebenfalls im Rahmen der vorliegenden Erfindung liegt. Wie insbesondere aus Figur 2 noch erkennbar ist, können die erzeugten Algen auch wiederum der Artemia-Produktion 4 als Nährstoff zugeführt werden.

Die bei der Umkehrosmose 7 gewonnenen Nährstoffe lassen sich ebenfalls für die Algen-Produktion 14 verwenden. Das gleiche gilt für die Rückstände der Filtration 6.

Wie ebenfalls in Figur 2 nochmals illustriert, kann die Prozessabwärme aus der Ethanolproduktion 1 in Form des Wärmeinhalts der Vinasse 2 bei Bezugszeichen 19 dazu verwendet werden, den Wärmebedarf der Artemia-Produktion 4 und/oder der Algen-Produktion 14 zumindest teilweise zu decken.

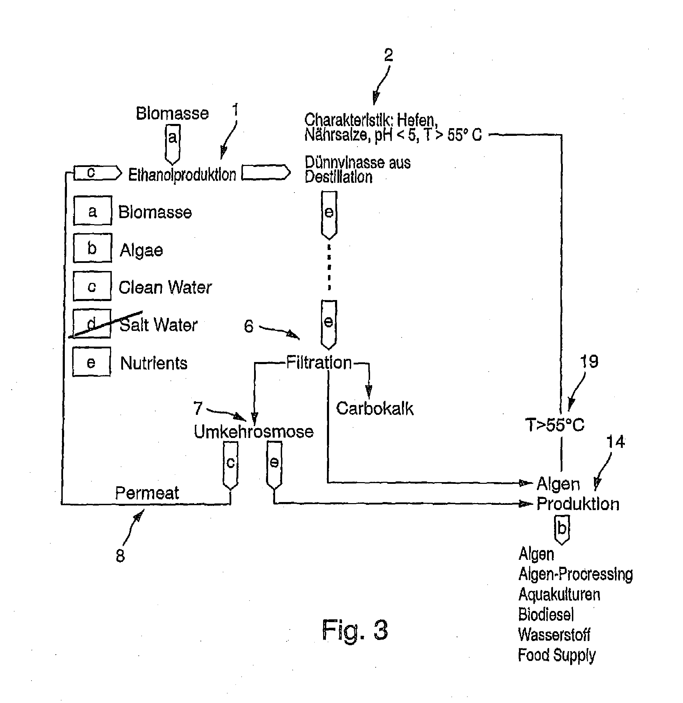

Figur 3 zeigt eine wiederum vereinfachte Ausgestaltung der Prozessabläufe gemäß Figur 2. Auch hier bezeichnen gleiche Bezugszeichen gleiche oder gleichwirkende Prozessschritte.

Gemäß der Darstellung in Figur 3 wird auf die Artemia-Produktion verzichtet. Nach erfolgter Filtration 6 der Vinasse 2 erfolgt eine Bereitstellung von Carbokalk (vgl. Figur 2) und die Versorgung der Algenproduktion 14.

Eine der Filtration 6 nachgeschaltete Umkehrosmose 7 liefert wiederum aufbereitetes Prozesswasser 8 für die Ethanolproduktion 1 bzw. Nährstoffe für die Algenproduktion 14.

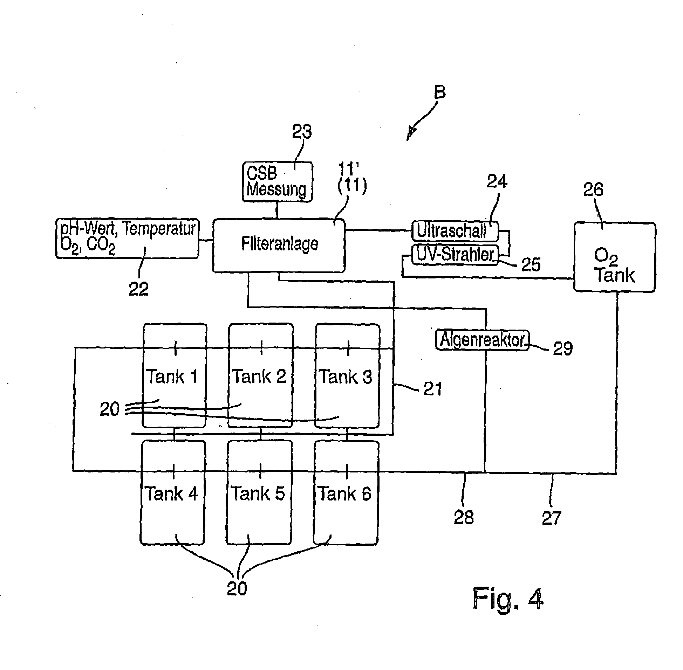

Figur 4 zeigt schematisch und im Detail eine Artemia-Produktionsanlage, wie sie im Rahmen der vorliegenden Erfindung Verwendung finden kann und weiter oben unter Bezugnahme auf die Figur 1 bei Bezugszeichen B bereits angesprochen wurde.

Die in Figur 4 dargestellte Anlage für die Artemia-Produktion gemäß Bezugszeichen 4 in Figur 1 umfasst eine Reihe von Aufzuchtbecken oder Tanks 20, je nach Größe bzw. Dimensionierung der Anlage. Die Tanks 20 sind mit Salzwasser gefüllt, um darin Krebstiere der Gattung Artemia (auch Salzkrebschen oder Salzwasserkrebse genannt) zu züchten, was dem Fachmann an sich bekannt ist. Gefüttert werden die Artemia zumindest teilweise mit chemisch aufbereiteter Dünnvinasse, wie weiter oben anhand von Figur 1 bereits ausführlich beschrieben (vergleiche dort Verfahrensschritte 3 und 4).

Vinasse ist protein- und nährstoffreich und eignet sich nach entsprechender Anhebung und Pufferung des pH-Werts hervorragend für die Verfütterung an Artemia. Insbesondere enthält Vinasse große Mengen an Hefezellen einer Größe von nur etwa 5 bis 10 µm, welche durch Filtration nicht entfernt werden können, Artemia jedoch als Nahrung dienen und so wenigstens teilweise beseitigt werden können.

Das mit Rückständen aus der Artemia-Produktion bzw. verbleibenden Bestandteilen der Vinasse belastete Ab- oder Prozesswasser gelangt über eine Leitung 21 mittels geeigneter Fördermittel (nicht gezeigt) zu einer Filteranlage 11', welche im Wesentlichen dem Bezugszeichen 11 in Figur 1 entspricht. Mit der Filteranlage 11', in Wirkverbindung stehen erste Messmittel 22 zur Bestimmung von pH-Wert, Temperatur, Sauerstoff- und CO₂-Gehalt des Prozesswassers. Weiterhin sind mit der Filteranlage 11' zweite Messmittel 23 verbunden, welche zur Durchführung einer CSB-Messung ausgebildet sind. Unter der Abkürzung "CSB" wird der so genannte chemische Sauerstoffbedarf verstanden, worunter man die Menge (volumenbezogene Masse) an Sauerstoff versteht, die zur vollständigen Oxidation der organischen und anorganischen Stoffe im Abwasser benötigt wird. Dies ist beispielsweise aus der kommunalen und industriellen Abwasseraufbereitung hinreichend bekannt. Weitere Wasserinhaltsstoffe, die in diesem Zusammenhang vorteilhafter Weise - auch im Rahmen der vorliegenden Erfindung - gemessen werden, sind Ammonium, Gesamtstickstoff, freies und Gesamtchlor, Nitrat und Phosphor. Entsprechende Messgeräte sind dem Fachmann hinreichend bekannt.

Weiterhin weist die Anlage bei Bezugszeichen 24 Ultraschallmittel auf, die dazu ausgebildet sind, bestimmte Belastungen des Prozesswassers aus der Artemia-Produktion durch Beschallung aufzuschließen und oder zu beseitigen. Die Ultraschallmittel 24 umfassen insbesondere geeignete Ultraschallschwinger mitsamt entsprechender Steuer-/Versorgungselektronik, was in Figur 4 aus Gründen der Übersichtlichkeit jedoch nicht explizit dargestellt ist. Grundsätzlich können alle Arten bekannter Ultraschallschwinger in diesem Zusammenhang Verwendung finden. Ein bevorzugter Frequenzbereich beim Betrieb der Ultraschallschwinger liegt im Megaschallbereich (f ≥ 500 kHz), um auf diese Weise insbesondere bakteriologische Belastungen des Prozesswassers zu beseitigen (Abtötung durch Zerstörung von Zellmembranen oder dergleichen).

Den Ultraschallmitteln 24 ist eine UV-Bestrahlungseinheit 25 zugeordnet, welche ebenfalls dazu vorgesehen und ausgebildet ist, biologische Belastungen des Prozesswassers durch Einwirkung kurzwelliger Lichtstrahlung, vorzugsweise im Bereich von 1 - 380 nm, vorzugsweise etwa 250 nm, zu beseitigen bzw. abzutöten.

Den UV-Bestrahlungsmitteln 25 ist ein Sauerstofftank 26 nachgeschaltet, welcher die Funktion hat, bei der Ultraschallbehandlung ausgegasten Sauerstoff zu ersetzen, um eine optimale Artemia-Aufzucht zu garantieren. Das so aufbereitete Prozesswasser gelangt über Leitungen 27, 28 zurück in die Tanks 20.

Wie der Fachmann erkennt, müssen UV-Behandlung und Ultraschallbehandlung nicht nacheinander bzw. in getrennten Vorrichtungen erfolgen, sondern können auch im wesentlichen gleichzeitig bzw. am selben Ort vorgenommen werden.

Zusätzlich umfasst die Anlage noch einen Algenreaktor 29, welcher zwischen die Filteranlage 11' und die Prozesswasser-Zuführleitungen 27, 28 geschaltet ist (vgl. Bezugszeichen 30). Der Algenreaktor 29 dient ebenfalls dazu, dass Prozesswasser der Artemia-Produktion von bestimmten Schad- und Abfallstoffen zu reinigen, was aus Algen-Klärwerken zur Abwasserreinigung bereits hinreichend bekannt ist. Derartige Algenreaktoren sind auch unter den Bezeichnungen Algen-Photobioreaktor zur Kohlendioxid-Fixierung oder zur Algen-Biomasse-Produktion bekannt.

Alternativ oder zusätzlich können mittels des Algenreaktors 29 gezielt Algen produziert werden. Die so gewonnenen Algen können entweder als Nahrungsmittel genutzt werden, beispielsweise wieder für die Artemia-Produktion (vgl. Bezugszeichen 4 und 15 in Figur 1) oder zur Erzeugung von Biodiesel, wie bereits angesprochen.

Die Verwendung von Ozon (O₃) zur Entkeimung des Prozesswassers ist ebenfalls möglich, sofern eine Schadeinwirkung auf die Artemia-Kultur insgesamt ausgeschlossen werden kann.

Die vorliegende Erfindung ermöglicht insbesondere die Artemia-Zucht und somit die Verwertung von Vinasse im großindustriellen Maßstab nach Art einer Intensivkultur. In diesem Zusammenhang stellt die Aufbereitung und Entkeimung des in der Artemia-Kultur anfallenden Prozesswassers eine besondere Herausforderung dar, wobei den Ultraschallmitteln 24 gemäß Figur 4 eine Schlüsselstellung zukommt. Ohne den hiermit erreichbaren Aufschluss der insbesondere biologischen Belastungen des Prozesswassers wäre das Verfahren in der Praxis nicht oder nur schwer umsetzbar bzw. in seinem Durchsatz und Ertrag auf nur kleine Mengen begrenzt, welche in keinem Verhältnis zu den insbesondere bei der Bioethanolproduktion anfallenden Vinassemengen stünden.

Figur 5 zeigt schematisch eine andere Krebstier-Produktionsanlage 100, wie sie bei der Durchführung des erfindungsgemäßen Verfahrens zum Einsatz kommen kann. In dem eigentlichen Kulturbehälter 101 erfolgt die Aufzucht von Copepoden (bei Bezugszeichen 102 nicht maßstäblich exemplarisch dargestellt) unter Verwendung von Vinasse, wie beschrieben. Innerhalb des Behälters 101 ist ein aus Rohrleitungsabschnitten gebildetes Rahmenelement 103 angeordnet, welches eine Anzahl von Faserbündeln 104 umfasst, deren einzelne Fasern 104a als Hohlfasern ausgebildet sind und zugleich als Membranfilter fungieren (Porengröße s 2µm, vorzugsweise ≤ 1 µm). Bei Bezugszeichen 103a wird mittels eines Fördermittels (Pumpe) 105 Prozesswasser 106 aus dem Behälter 101 abgesaugt (was durch gestrichelte Pfeile symbolisiert ist) und dabei im Rahmen einer outside-in-Filtration durch die Membranfilter-Hohlfasern 104a gereinigt. Das entstehende Permeat, das mit Ammonium und anderen organischen Molekülen verunreinigt ist, gelangt von der Pumpe 105 entweder zu einem Biofilter 107, wo es bakteriell weiter aufbereitet wird, oder es wird bei Bezugszeichen 108 als Dünger für eine Produktion von (marinen) Algen eingesetzt, worauf weiter oben schon hingewiesen wurde. Das bei 107 gereinigte Permeat gelangt anschließend zurück in den Behälter 101.

Zur Reinigung der Membranfilter-Hohlfasern 104a wird das Rahmenelement 103 mitsamt den Faserbündeln 104 aus dem Behälter 101 entnommen (strichpunktierter Pfeil) und in den Tank 109 einer Ultraschallreinigungsvorrichtung 110 eingelegt, der mit einem Reinigungsmedium (-fluid) 111 gefüllt ist. Ein Ultraschallwandler 112 der Ultraschallreinigungsvorrichtung 110 beaufschlagt das Rahmenelement 103 mit Ultraschall 113, um die Membranfilter-Hohlfasern 104a von Verschmutzung zu befreien. Anschließend kann das Rahmenelement 103 wieder in den Behälter 101 eingesetzt werden.

Im Rahmen der vorliegenden Erfindung wird erstmalig ein Gesamtprozess vorgeschlagen, welcher der beschriebenen Vinasseproblematik umfassend entgegenwirken kann.

## Patentansprüche

1. Verfahren zur Produktion von Futtermittel aus Krebstieren der Gattung Artemia oder Copepoda oder Daphnia oder aus Kleinlebewesen in Form von Rotifera oder Protozoa, **dadurch gekennzeichnet, dass** die Krebstier-/Kleinlebewesen-Kulturen zumindest teilweise mit Vinasse, vorzugsweise Dünnvinasse, insbesondere aus der Bioethanolproduktion, und den darin enthaltenen Hefezellen gefüttert werden.

2. Verfahren zur Verarbeitung von Vinasse, insbesondere im Zuge der Bioethanolgewinnung, **dadurch gekennzeichnet, dass** die Vinasse als Futter für Krebstier-Kulturen der Gattung Artemia und/oder Copepoda und/oder Daphnia, für Kleinlebewesen-Kulturen zur Zucht von Rotifera oder Protozoa und/oder als Nährstoff für Algenkulturen, insbesondere marine Algenkulturen, verwendet wird, um in der Vinasse enthaltene Hefezellen zumindest teilweise zu entfernen.

3. Verfahren nach Anspruch 1 oder 2, **dadurch gekennzeichnet, dass** der pH-Wert der Vinasse vor dem Verfüttern, insbesondere an Artemia, Copepoda, Daphnia, Rotifera oder Protozoa, angehoben und vorzugsweise gepuffert wird.

4. Verfahren nach mindestens einem der Ansprüche 1 bis 3, **dadurch gekennzeichnet, dass** die Artemia, die Copepoda, die Daphnia, die Rotifera, die Protozoa- und/oder die Algen in geschlossenen Intensivkulturen gehalten werden.

5. Verfahren nach Anspruch 4, **dadurch gekennzeichnet, dass** das in den Krebstier-Kulturen, in den Kleinlebewesen-Kulturen und/oder den Algen-Kulturen verwendete Wasser zur Entkeimung mit Ultraschall beaufschlagt wird.

6. Verfahren nach Anspruch 4 oder 5, **dadurch gekennzeichnet, dass** das in den Krebstier-Kulturen, in den Kleirilebewesen-Kulturen und/oder den Algen-Kulturen verwendete Wasser zur Entkeimung mit kurzwelligem Licht, vorzugsweise im ultravioletten Spektralbereich, beaufschlagt wird.

7. Verfahren nach mindestens einem der Ansprüche 1 bis 6, **dadurch gekennzeichnet, dass** Prozesswasser aus den Krebstier-/Kleinlebewesen-Kulturen carbonatiert und, vorzugsweise mittels zusätzlicher Filtration und/oder Umkehrosmose, von Fremdstoffen getrennt wird.

8. Verfahren nach Anspruch 7, **dadurch gekennzeichnet, dass** die entstehenden Salze und/oder Salzlaugen als Düngemittel verwendet werden, insbesondere für Algen-Kulturen, höchst vorzugsweise marine Algen-Kulturen.

9. Verfahren nach Anspruch 8, **dadurch gekennzeichnet, dass** die gezüchteten Algen als Futtermittel für die Krebstier-/Kleinlebewesen-Kulturen verwendet werden, insbesondere für geschlossene Krebstier-/Kleinlebewesen-Intensivkulturen, oder als Rohstoff bzw. zur weiteren Verarbeitung, insbesondere zur Treibstofferzeugung.

10. Verfahren nach Anspruch 8 oder 9. **dadurch gekennzeichnet, dass** Prozesswasser aus den Algen-Kulturen carbonatiert und, vorzugsweise mittels zusätzlicher Filtration und/oder Umkehrosmose, von Fremdstoffen getrennt wird, und das höchst vorzugsweise die entstehenden Salze und/oder Salzlaugen wieder in die Krebstier-/Kleinlebewesen-Kulturen eingespeist werden.

11. Verfahren nach mindestens einem der Ansprüche 1 bis 10, **dadurch gekennzeichnet, dass** Prozesswasser aus den Krebstier-/Kleinlebewesen-Kulturen über Membranfilter gefiltert wird, welche Membranfilter vorzugsweise in einem Kulturbehälter der Krebstier-/Kleinlebewesen-Kulturen angeordnet sind,

12. Verfahren nach zumindest Anspruch 11, **dadurch gekennzeichnet, dass** für die Filtrierung Membranfilter-Hohlfasern verwendet werden, vorzugsweise Bündel von Hohlfasern, höchst vorzugsweise mit einer Porengröße < 2 µm, insbesondere ≤ 1 µm.

13. Verfahren nach zumindest Anspruch 11 oder 12, **dadurch gekennzeichnet, dass** die Filtrierung im outside-in-Verfahren durchgeführt wird, dass das Permeat aus dem Kulturbehälter nach außen abgeführt wird, insbesondere durch die Hohlfasern gemäß Anspruch 12, und dass das Permeat anschließend gereinigt wird, vorzugsweise mittels eines Biofilters, oder dass das Permeat als Dünger für Algenkulturen, insbesondere marine Algenkulturen, verwendet wird.

14. Verfahren nach zumindest einem der Ansprüche 11 bis 13, **dadurch gekennzeichnet, dass** die für die Filtrierung verwendeten Membranfilter aus dem Kulturbehälter entnommen werden, vorzugsweise als Teil einer insgesamt aus dem Kulturbehälter entnehmbaren Haltekonstruktion, und dass die entnommenen Membranfilter anschließend gereinigt werden, höchst vorzugsweise in einer Ultraschallreinigungsvorrichtung durch Beaufschlagung mit Ultraschall.

15. Verfahren zur Produktion von Futtermittel aus Krebstieren der Gattung Artemia oder Copepoda oder Daphnia oder aus Kleinlebewesen in Form von Rotifera oder Protozoa und/oder zur Produktion von Rohstoffen aus Algen, **dadurch gekennzeichnet, dass** bei der Vinasseproduktion, insbesondere im Zuge der Bioethanolgewinnung, erzeugte Prozesswärme zum Erwärmen von Wasser für die Krebstier-, Kleinlebewesen- und/oder Algenzucht verwendet wird, vorzugsweise zusätzlich **gekennzeichnet durch** die weiteren Merkmale nach einem der Ansprüche 1 bis 14.

## Claims

1. Method for the production of animal feedstuff from crustaceans of the genus Artemia or Copepoda or Daphnia or from micro-organisms in the form of rotifers or protozoa, **characterised in that** the crustacean/micro-organism cultures are fed at least partly with vinasse, preferably thin vinasse, especially from bioethanol production, and the yeast cells contained therein.

2. Method of processing vinasse, especially in the course of bioethanol production, **characterised in that** the vinasse is used as feed for crustacean cultures of the genus Artemia and/or Copepoda and/or Daphnia, for micro-organism cultures for cultivating rotifers or protozoa and/or as nutrient for algae cultures, especially marine algae cultures, in order to remove at least to some extent yeast cells contained in the vinasse.

3. Method according to claim 1 or 2, **characterised in that** the pH value of the vinasse is raised and preferably buffered before feeding, especially to Artemia, Copepoda, Daphnia, rotifers or protozoa.

4. Method according to at least one of claims 1 to 3, **characterised in that** the Artemia, the Copepoda, the Daphnia, the rotifers, the protozoa and/or the algae are kept in closed intensive cultures.

5. Method according to claim 4, **characterised in that** the water used in the crustacean cultures, in the micro-organism cultures and/or the algae cultures is subjected to ultrasound for the purpose of sterilisation.

6. Method according to claim 4 or 5, **characterised in that** the water used in the crustacean cultures, in the micro-organism cultures and/or the algae cultures is exposed to short-wave light, preferably in the ultraviolet spectral range, for the purpose of sterilisation.

7. Method according to at least one of claims 1 to 6, **characterised in that** process water from the crustacean/micro-organism cultures is subjected to carbonatation and is separated from impurities, preferably by means of additional filtration and/or reverse osmosis.

8. Method according to claim 7, **characterised in that** the resulting salts and/or brines are used as fertiliser, especially for algae cultures, most preferably marine algae cultures.

9. Method according to claim 8, **characterised in that** the cultivated algae are used as feedstuff for the crustacean/micro-organism cultures, especially for closed intensive crustacean/micro-organism cultures, or as raw material or for further processing, especially for fuel production.

10. Method according to claim 8 or 9, **characterised in that** process water from the algae cultures is subjected to carbonatation and is separated from impurities, preferably by means of additional filtration and/or reverse osmosis, and most preferably the resulting salts and/or brines are fed back into the crustacean/microorganism cultures.

11. Method according to at least one of claims 1 to 10, **characterised in that** process water from the crustacean/micro-organism cultures is filtered through membrane filters, which membrane filters are preferably arranged in a culture container of the crustacean/micro-organism cultures.

12. Method according to at least claim 11, **characterised in that** for the filtering there are used hollow fibre membrane filters, preferably bundles of hollow fibres, most preferably having a pore size < 2 µm, especially 1 µm.

13. Method according to at least claim 11 or 12, **characterised in that** the filtering is carried out in an outside-in process, the permeate is discharged from the culture container to the outside, especially through the hollow fibres according to claim 12, and the permeate is then purified, preferably by means of a biofilter, or the permeate is used as fertiliser for algae cultures, especially marine algae cultures.

14. Method according to at least one of claims 11 to 13, **characterised in that** the membrane filters used for the filtering are removed from the culture container, preferably as part of a holding structure removable as a whole from the culture container, and the removed membrane filters are then cleaned, most preferably in an ultrasound purification device by subjection to ultrasound.

15. Method for the production of animal feedstuff from crustaceans of the genus Artemia or Copepoda or Daphnia or from micro-organisms in the form of rotifers or protozoa and/or for the production of raw materials from algae, **characterised in that** process heat generated in vinasse production, especially in the course of bioethanol production, is used for heating water for the crustacean, micro-organism and/or algae cultivation, preferably additionally **characterised by** the further features according to any one of claims 1 to 14.

## Revendications

1. Procédé de production d'une substance d'alimentation animale à partir de crustacés du genre artémies, copépodes ou daphnies, voire à partir de petits organismes revêtant la forme de rotifères ou de protozoaires, **caractérisé par le fait que** les cultures de crustacés/petits organismes sont nourries au moins partiellement par de la vinasse, de préférence de la vinasse diluée provenant notamment de la production de bioéthanol, et par les cellules de levures qu'elle contient.

2. Procédé de traitement de la vinasse, en particulier au cours de la production de bioéthanol, **caractérisé par le fait que** la vinasse est utilisée en tant que nutriment pour cultures de crustacés du genre artémies et/ou copépodes et/ou daphnies, pour cultures de petits organismes dédiées à l'élevage de rotifères ou de protozoaires, et/ou en tant que substance nutritive pour cultures d'algues, notamment des cultures d'algues marines, afin d'éliminer au moins partiellement des cellules de levures contenues dans ladite vinasse.

3. Procédé selon la revendication 1 ou 2, **caractérisé par le fait que** le pH de la vinasse est augmenté et, de préférence, tamponné préalablement à l'apport de nutriment à des artémies, des copépodes, des daphnies, des rotifères ou des protozoaires en particulier.

4. Procédé selon au moins l'une des revendications 1 à 3, **caractérisé par le fait que** les artémies, les copépodes, les daphnies, les rotifères, les protozoaires et/ou les algues sont maintenu(e)s dans des cultures intensives en milieu fermé.

5. Procédé selon la revendication 4, **caractérisé par le fait que** l'eau, utilisée dans les cultures de crustacés, dans les cultures de petits organismes et/ou dans les cultures d'algues, est soumise à l'action d'ultrasons en vue de la stérilisation.

6. Procédé selon la revendication 4 ou 5, **caractérisé par le fait que** l'eau utilisée dans les cultures de crustacés, dans les cultures de petits organismes et/ou dans les cultures d'algues est soumise, en vue de la stérilisation, à l'action d'une lumière à ondes courtes située, de préférence, dans la zone spectrale ultraviolette.

7. Procédé selon au moins l'une des revendications 1 à 6, **caractérisé par le fait que** de l'eau de processus émanant des cultures de crustacés/petits organismes est carbonatée et est, de préférence, séparée d'avec des corps étrangers par filtration supplémentaire et/ou par osmose inverse.

8. Procédé selon la revendication 7, **caractérisé par le fait que** les sels et/ou les saumures résultant(e)s sont utilisé(e)s en tant qu'engrais pour des cultures d'algues, en particulier, et pour des cultures d'algues marines avec préférence maximale.

9. Procédé selon la revendication 8, **caractérisé par le fait que** les algues élevées sont utilisées en tant que nutriments pour les cultures de crustacés/petits organismes, notamment pour des cultures intensives de crustacés/petits organismes en milieu fermé, voire, respectivement, en tant que matière première ou en vue d'un traitement ultérieur, en particulier pour la production de carburant.

10. Procédé selon la revendication 8 ou 9, **caractérisé par le fait que** de l'eau de processus émanant des cultures d'algues est carbonatée et est, de préférence, séparée d'avec des corps étrangers par filtration supplémentaire et/ou par osmose inverse ; et **par le fait que** les sels et/ou les saumures résultant(e)s sont réintroduit(e)s, avec préférence maximale, dans les cultures de crustacés/petits organismes.

11. Procédé selon au moins l'une des revendications 1 à 10, **caractérisé par le fait que** de l'eau de processus émanant des cultures de crustacés/petits organismes est filtrée par l'intermédiaire de filtres à membranes, lesquels filtres à membranes sont disposés, de préférence, dans un récipient de culture desdites cultures de crustacés/petits organismes.

12. Procédé selon au moins la revendication 11, **caractérisé par le fait que** des fibres creuses pour filtres à membranes, de préférence des faisceaux de fibres creuses présentant une grosseur de pores < 2 µm, et notamment ≤ 1 µm avec préférence maximale, sont utilisé(e)s en vue de la filtration.

13. Procédé selon au moins la revendication 11 ou 12, **caractérisé par le fait que** la filtration est effectuée suivant le procédé s'opérant de l'extérieur vers l'intérieur ; **par le fait que** le perméat est évacué vers l'extérieur à partir du récipient de culture, en particulier par l'intermédiaire des fibres creuses conformes à la revendication 12 ; et **par le fait que** ledit perméat est ensuite épuré, de préférence au moyen d'un filtre biologique ; ou **par le fait que** ledit perméat est utilisé en tant qu'engrais pour cultures d'algues, en particulier des cultures d'algues marines.

14. Procédé selon au moins l'une des revendications 11 à 13, **caractérisé par le fait que** les filtres à membranes utilisés pour la filtration sont enlevés du récipient de culture, de préférence en tant que partie intégrante d'une structure de maintien pouvant être intégralement enlevée dudit récipient de culture ; et **par le fait que** lesdits filtres à membranes enlevés sont ensuite nettoyés, avec préférence maximale, par exposition à des ultrasons dans un dispositif de nettoyage par ultrasons.

15. Procédé dévolu à la production d'une substance d'alimentation animale à partir de crustacés du genre artémies, copépodes ou daphnies, voire à partir de petits organismes revêtant la forme de rotifères ou de protozoaires, et/ou à la production de matières premières à partir d'algues, **caractérisé par le fait que** de la chaleur de processus engendrée lors de la production de vinasse, en particulier au cours de la production de bioéthanol, est utilisée pour chauffer de l'eau dédiée à l'élevage de crustacés, de petits organismes et/ou d'algues, additionnellement caractérisé, de préférence, par les autres caractéristiques conformes à l'une des revendications 1 à 14.
